# EUROPÄISCHE PATENTSCHRIFT

(11) **EP 2 173 756 B1**
(45) Veröffentlichungstag und Bekanntmachung des Hinweises auf die Patenterteilung: **16.03.2011**
(21) Anmeldenummer: 08784525.1
(22) Anmeldetag: 20.06.2008
(51) Int. Cl.: C07D 498/04, A61K 31/395, A61P 31/10

(54) **MAKROLAKTON-DERIVATE**
MACROLACTONE DERIVATIVES
DÉRIVÉS DE MACROLACTONE

(30) Priorität: 04.07.2007 EP 07290841
(43) Veröffentlichungstag der Anmeldung: 14.04.2010
(73) Patentinhaber: Sanofi-Aventis, 75013 Paris (FR)
(72) Erfinder: HOFFMANN, Holger, 65926 Frankfurt am Main (DE); KLEMKE-JAHN, Christine, 65926 Frankfurt am Main (DE); SCHUMMER, Dietmar, 65926 Frankfurt am Main (DE); KOGLER, Herbert, 65926 Frankfurt am Main (DE)
(74) Vertreter: Sieber, Frank
(86) Internationale Anmeldenummer: PCT/EP2008/004971
(87) Internationale Veröffentlichungsnummer: WO 2009/003595

(56) Entgegenhaltungen:
- WO-A-01/07439
- WO-A-2006/100330

## Beschreibung

Die vorliegende Erfindung betrifft neue Makrolaktone, Verfahren zu ihrer Herstellung und Verwendung. Es wurde nun gefunden, dass der Mikroorganismenstamm ST 201196 (DSM 18870), neuartige Makrozyklen zu bilden vermag, die eine stark antifungische Aktivität gegen den Pilz *Candida albicans* aufweisen. Die Verbindungen sind demzufolge für die Behandlung von lokalen und/oder systemischen Pilzerkrankungen geeignet.

Zur Behandlung von Infektionskrankheiten wird eine große Zahl von Antiinfektiva therapeutisch eingesetzt. Die Krankheitserreger werden aber zunehmend resistent gegen die verwendeten Arzneimittel, es droht sogar eine große Gefahr durch sogenannte multiresistente Keime, die nicht nur gegen einzelne Antiinfektivagruppen, sondern gleichzeitig mehrere Resistenzen tragen. Es gibt sogar Krankheitserreger, die gegen alle im Handel erhältlichen Antiinfektiva resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antünfektiva beschrieben worden, die meisten sind jedoch zu toxisch, um als Arzneimittel eingesetzt werden zu können.

Die vorliegende Erfindung betrifft eine Verbindung der Formel (I), 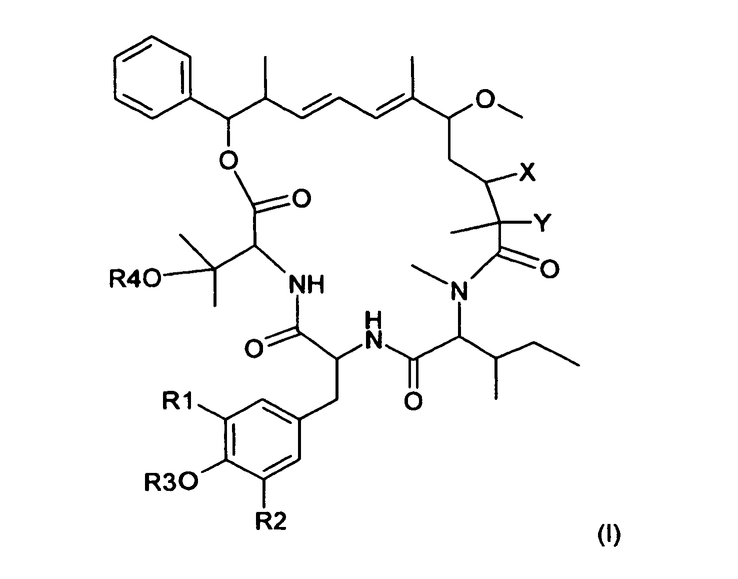 worin
X und Y unabhängig voneinander OH, O-(C₁-C₆)-Alkyl, NH₂ oder NH-(C₁-C₆)-Alkyl sind, oder X und Y zusammen eine Gruppe -O- bilden,
R1 und R2 unabhängig voneinander Cl oder H sind,
R3 H, (C₁-C₆)-Alkyl, C(=O)-(C₁-C₆)-Alkyl oder (C₁-C₆)-Alkylen-NH-(C₁-C₆)-alkyl bedeutet, und
R4 H, (C₁-C₆)-Alkyl oder C(=O)-(C₁-C₆)-Alkyl bedeutet,
oder ein physiologisch verträgliches Salz einer Verbindung der Formel (I).

Bevorzugt betrifft die Erfindung eine Verbindung der Formel (I), worin X und Y zusammen eine Gruppe -O- bilden, folglich bilden X und Y in der entsprechenden bevorzugten Verbindung zusammen mit den C-Atomen an die sie gebunden sind eine Epoxidgruppe.

Ferner bevorzugt betrifft die Erfindung eine Verbindung der Formel (I), worin R1 und R2 Cl sind.

Ferner bevorzugt betrifft die Erfindung eine Verbindung der Formel (I), worin R1 gleich Cl und R2 gleich H ist.

R3 und R4 sind vorzugsweise unabhängig voneinander H, (C₁-C₆)-Alkyl oder C(=O)-(C₁-C₆)-Alkyl, besonders bevorzugt sind beide R3 und R4 gleich H.

Besonders bevorzugt betrifft die Erfindung eine Verbindung der Formel (1), worin X und Y zusammen eine Gruppe -O- bilden, R1 und R2 unabhängig voneinander Cl oder H sind, und R3 und R4 unabhängig voneinander H, (C₁-C₆)-Alkyl oder C(=O)-(C₁-C₆)-Alkyl bedeuten,
ferner eine Verbindung der Formel (I), worin X und Y zusammen eine Gruppe -O-bilden, R1 und R2 gleich Cl sind und R3 und R4 unabhängig voneinander H, (C₁-C₆)-Alkyl oder C(=O)-(C₁-C₆)-Alkyl bedeuten,
ferner eine Verbindung der Formel (I), worin X und Y zusammen eine Gruppe -O-bilden, R1 gleich Cl, R2 gleich H, und R3 und R4 unabhängig voneinander H, (C₁-C₆)-Alkyl oder C(=O)-(C₁-C₆)-Alkyl bedeuten,
ferner eine Verbindung der Formel (I), worin
X und Y zusammen eine Gruppe -O- bilden,
R3 und R4 gleich H sind, und
worin R1 und R2 unabhängig voneinander gleich Cl oder H sind,
vorzugsweise worin R1 und R2 gleich Cl (im folgenden auch als Verbindung der Formel (II) bezeichnet), 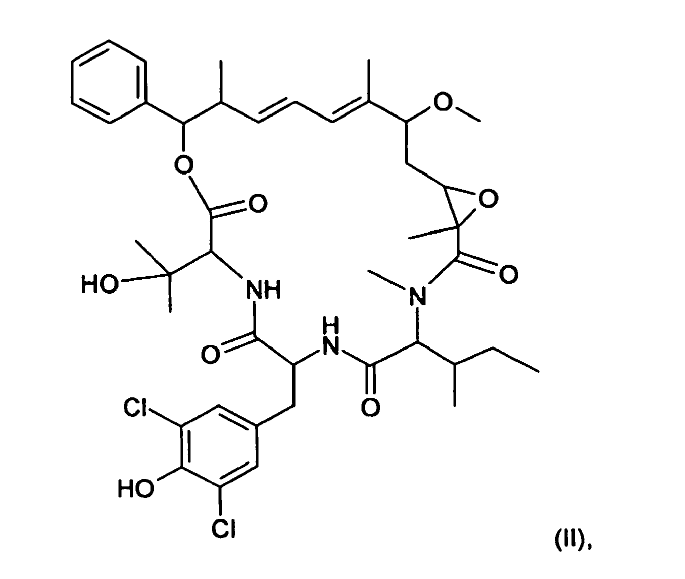 oder worin ferner vorzugsweise R1 gleich Cl und R2 gleich H sind (im folgenden auch als Verbindung der Formel (III) bezeichnet), 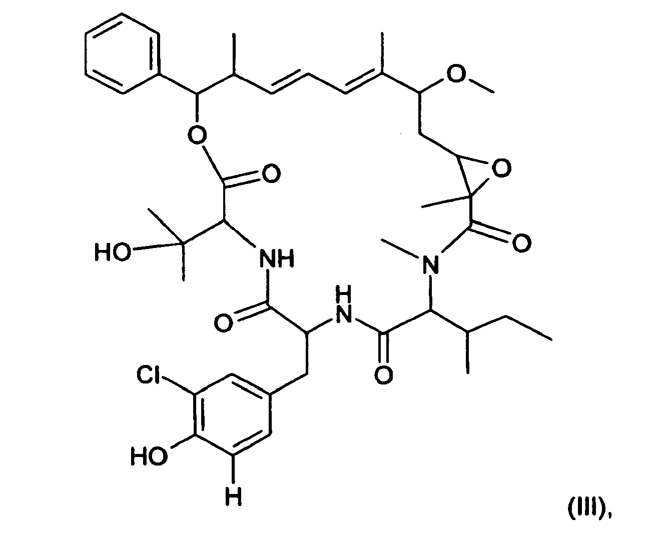 oder worin ferner vorzugsweise R1 und R2 gleich H sind.

(C₁-C₆)-Alkyl bedeutet eine geradkettige oder verzweigte Alkylgruppe mit 1 bis 6 Kohlenstoffatomen, beispielsweise Methyl, Ethyl, n-Propyl, iso-Propyl, n-Butyl, sec-Butyl, tert-Butyl, n-Pentyl oder n-Hexyl.

Chiralitätszentren in den Verbindungen der Formel (I) können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diastereomerengemische.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die vorliegende Erfindung betrifft weiterhin alle offensichtlichen chemischen Äquivalenten der erfindungsgemäßen Verbindungen der Formel (I). Solche Äquivalente sind Verbindungen, die einen geringfügigen chemischen Unterschied aufweisen, also die gleiche Wirkung haben oder sich unter milden Bedingungen in die efindungsgemäßen Verbindungen umwandeln. Zu den genannten Äquivalenten gehören z.B. auch Salze, Reduktionsprodukte, Oxidationsprodukte, Ester, Ether, Acetale oder Amide der Verbindungen der Formel (I) sowie Äquivalente die der Fachmann mit Standardmethoden herstellen kann.

Die Erfindung betrifft ferner ein Verfahren zur Herstellung einer Verbindung der Formel (I), 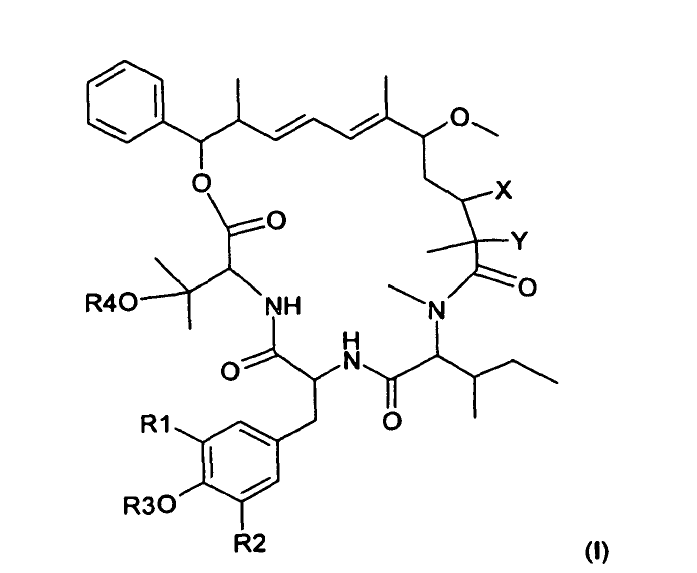 worin
X und Y unabhängig voneinander OH, O-(C₁-C₆)-Alkyl, NH₂ oder NH-(C₁-C₆)-Alkyl sind, oder X und Y zusammen eine Gruppe -O- bilden,
R1 und R2 unabhängig voneinander Cl oder H sind,
R3 H, (C₁-C₆)-Alkyl, C(=O)-(C₁-C₆)-Alkyl oder (C₁-C₆)-Alkylen-NH-(C₁-C₆)-alkyl bedeutet, und
R4 H, (C₁-C₆)-Alkyl oder C(=O)-(C₁-C₆)-Alkyl bedeutet,
oder eines physiologisch verträgliches Salz einer Verbindung der Formel (I),
dadurch gekennzeichnet, dass
1. der Stamm ST 201196 (DSM 18870) oder einer seiner Varianten und/oder Mutanten unter geeigneten Bedingungen in einem Kulturmedium fermentiert wird, das eine Cl-Quelle enthält, bis sich eine oder mehrere der Verbindungen der Formel (I) in dem Kulturmedium anhäuft und
2. eine Verbindung der Formel (I) aus dem Kulturmedium isoliert wird, und
3. die Verbindung der Formel (I) gegebenenfalls derivatisiert und/oder in ein physiologisch verträgliches Salz umgewandelt wird.

Das Kulturmedium ist eine Nährlösung oder ein Festmedium mit mindestens einer Kohlenstoff- und Stickstoffquelle sowie den üblichen anorganischen Salzen.

Als Cl-Quelle kann beispielsweise NaCl oder CaCl₂ verwendet werden. In diesem Fall produziert der Stamm ST 201196 (DSM 18870) vorzugsweise Verbindungen der Formel (I), in denen beide Reste R1 und R2 gleich Cl sind oder in denen R gleich Cl und R2 gleich H ist. Bevorzugt betrifft die Erfindung ein Verfahren zur Herstellung einer Verbindung der Formel (I), wobei R1 und R2 gleich Cl sind. Ferner betrifft die Erfindung bevorzugt ein Verfahren zur Herstellung einer Verbindung der Formel (I), wobei R1 gleich H, R2 gleich Cl ist.

Bevorzugt betrifft die Erfindung ein Verfahren zur Herstellung einer Verbindung der Formel (I), worin X und Y eine Gruppe -O- bilden, und ferner R3 und R4 wie oben beschrieben oder bevorzugt H sind.

Besonders bevorzugt betrifft die Erfindung ein Verfahren zur Herstellung einer Verbindung der Formel (II). Ferner besonders bevorzugt betrifft die Erfindung ein Verfahren zur Herstellung einer Verbindung der Formel (III). Ferner betrifft die Erfindung besonders bevorzugt ein Verfahren zur Herstellung einer Verbindung der Formel (I), wobei R1, R2, R3 und R4 gleich H sind.

Das erfindungsgemäße Verfahren kann für die Fermentation im Labormaßstab (Milliliter- bis Litermaßstab) und für den industriellen Maßstab (Kubikmetermaßstab) eingesetzt werden.

Als Kohlenstoffquellen für die Fermentation eignen sich assimilierbare Kohlenhydrate und Zuckeralkohole, wie Glukose, Laktose, Saccharose oder D-Mannitol sowie kohlenhydrathaltige Naturprodukte, wie z.B. Malzextrakt oder Hefeextrakt. Als stickstoffhaltige Nährstoffe kommen in Betracht: Aminosäuren; Peptide und Proteine sowie deren Abbauprodukte, beispielsweise Probion F (Applied Microbiology and Biotechnology 1984, 19(1), 23-28), Casein, Pepton oder Trypton; Fleischextrakte; Hefeextrakte; Gluten; gemahlene Samen, beispielsweise von Mais, Weizen, Bohnen, Soja oder der Baumwollpflanze; Destillationsrückstände der Alkoholherstellung; Fleischmehle; Hefeextrakte; Ammoniumsalze; Nitrate. Vorzugsweise ist die Stickstoffquelle ein oder mehrere synthetisch bzw. biosynthetisch gewonnene Peptide. Anorganische Salze sind beispielsweise Chloride, Carbonate, Sulfate oder Phosphate der Alkali- oder Erdalkalimetalle, Eisen, Zink, Kobalt und Mangan. Spurenelemente sind beispielsweise Kobalt und Mangan.

Geeignete Bedingungen für die Bildung der erfindungsgemäßen Substanzen sind wie folgt: Die Bildung der erfindungsgemäßen Substanzen verläuft bevorzugt in einem Kulturmedium, das entweder 0,05 bis 5 %, bevorzugt 0,1 bis 2,5 % Probion F; 0,02 bis 1,0 %, bevorzugt 0,05 bis 0,5 % CaCl₂ x 2 H₂O; 0,02 bis 1,5 %, bevorzugt 0,05 bis 0,7 % MgSO₄ x 7 H₂O sowie 0,00001 % bis 0,001 % Cyanocobalamin enthält, 1-5 % des Adsorberharzes XAD-16, oder 0,05 bis 5 %, bevorzugt 0,1 bis 2,5 % Hafermehl; 0,2 bis 5,0 %, bevorzugt 0,1 bis 2 % Glycerin; 0,02 bis 1,0 %, bevorzugt 0,05 bis 0,5 % CaCl₂; 0,02 bis 1,5%, bevorzugt 0,05 bis 0,7 % MgSO₄ x 7 H₂O sowie 0,00001 % bis 0,001 % Cyanocobalamin enthält. Die Angaben in Prozent sind jeweils bezogen auf das Gewicht der gesamten Nährlösung.

Die Kultivierung des Mikroorganismus erfolgt aerob, also beispielsweise submers unter Schütteln oder Rühren in Schüttelkolben oder Fermentern oder auf Festmedium, gegebenenfalls unter Einführen von Luft oder Sauerstoff. Sie kann in einem Temperaturbereich von etwa 18 bis 35°C, vorzugsweise bei etwa 20 bis 32°C, insbesondere bei 27 bis 30°C durchgeführt werden. Der pH-Bereich sollte zwischen 4 und 10 liegen, vorzugsweise zwischen 6.5 und 9. Man kultiviert den Mikroorganismus unter diesen Bedingungen im allgemeinen über einen Zeitraum von 3 bis 18 Tagen, vorzugsweise 144 bis 216 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10 bis 1:100, überimpft werden. Die Vorkultur erhält man z. B., indem man den Stamm in Form von vegetativen Zellen oder Fruchtkörpern in eine Nährlösung überimpft und etwa 3 bis 13 Tage, bevorzugt 96 bis 240 Stunden, wachsen lässt. Vegetative Zellen und/oder Fruchtkörper können beispielsweise erhalten werden, indem man den Stamm etwa 3 bis 15 Tage, bevorzugt 7 bis 10 Tage, auf einem festen oder flüssigen Nährboden, beispielsweise Hefeagar wachsen lässt. Die Isolierung bzw. Aufreinigung der Substanzen der Formel (I) aus dem Kulturmedium erfolgt nach bekannten Methoden unter Berücksichtigung der chemischen, physikalischen und biologischen Eigenschaften der Naturstoffe. Zum Testen der Konzentration der jeweiligen Derivate im Kulturmedium oder in den einzelnen Isolierungsstufen wurde HPLC verwendet.

Zur Isolierung wird die Kulturbrühe zentrifugiert und/oder über eine Nutsche abfiltriert. Das Mycel mit dem XAD wird lyophilisiert, anschließend werden die Naturstoffe vom Lyophilisat mit einem organischen Lösungsmittel, beispielsweise Methanol oder 2-Propanol extrahiert. Die organische Lösungsmittelphase enthält die erfindungsgemäßen Naturstoffe, sie wird gegebenenfalls im Vakuum konzentriert und weiter aufgereinigt.

Die weitere Aufreinigung einer oder mehrerer erfindungsgemäßer Verbindungen erfolgt durch Chromatographie an geeigneten Materialien, vorzugsweise z. B. an Molekularsieben, an Kieselgel, Aluminiumoxid, an Ionenaustauschern oder an Adsorberharzen bzw. an Umkehrphasen (reversed phase, RP). Mit Hilfe dieser Chromatographie werden die Naturstoff-Derivate getrennt. Die Chromatographie der erfindungsgemäßen Verbindungen erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, 2-Propanol und Acetonitril, in einer Konzentration von 0 bis 100 % Lösungsmittel oder auch alle gepufferten wässrigen Lösungen, die mit organischen Lösemitteln mischbar sind. Die zu verwendenden Puffer sind die gleichen wie oben angegeben.

Die Trennung der erfindungsgemäßen Verbindungen aufgrund ihrer unterschiedlichen Polarität erfolgt mit Hilfe der Reversed Phase Chromatographie, beispielsweise an MCl® (Adsorberharz, Mitsubishi, Japan) oder Amberlite XAD® (TOSOHAAS), oder an einem anderen hydrophoben Material, wie zum Beispiel an RP-8- oder RP-18-Phasen. Außerdem kann die Trennung mit Hilfe der Normalphasen-Chromatographie, zum Beispiel an Kieselgel, Aluminiumoxid erfolgen.

Die Chromatographie der Naturstoff-Derivate erfolgte nach dem Fachmann bekannten Methoden, vorzugsweise mit gepufferten, basischen oder angesäuerten wässrigen Lösungen oder Gemischen von wässrigen Lösungen mit Alkoholen oder anderen, mit Wasser mischbaren organischen Lösemitteln. Als organisches Lösemittel wird vorzugsweise Acetonitril und/oder Methanol verwendet.

Unter gepufferten, basischen oder angesäuerten wässrigen Lösungen versteht man z.B. Wasser, Phosphatpuffer, Ammoniumacetat, Ammoniumformiat, Citratpuffer in einer Konzentration von bis zu 0,5 M sowie Ameisensäure, Essigsäure, Trifluoressigsäure, Ammoniak, Triethylamin oder allen handelsüblichen, dem Fachmann bekannten Säuren und Basen, vorzugsweise in einer Konzentration von bis zu 1 %. Bei gepufferten wässrigen Lösungen wird 0,1 % Ammoniumacetat besonders bevorzugt.

Chromatographiert wurde beispielsweise mit einem Gradienten, der mit 100 % Wasser begann und mit 100 % Lösemittel endete, vorzugsweise wurde ein linearer Gradient von 5 bis 95 % Acetonitril gefahren.

Alternativ kann auch eine Gelchromatographie oder die Chromatographie an hydrophoben Phasen erfolgen. Die Gelchromatographie wird an Polyacrylamid- oder Mischpolymergelen durchgeführt, wie z.B. Biogel-P 2® (Biorad) oder Fractogel TSK HW 40® (Merck, Deutschland). Die Reihenfolge der vorgenannten Chromatographien ist umkehrbar.

Sofern die Verbindung der Formel (I) als Stereoisomerengemisch vorliegt, können die Stereoisomere über bekannte Methoden getrennt werden, beispielsweise durch Trennung über eine chirale Säule.

Die Derivatisierung der OH-Gruppen an der 3,5-Dichlor-Tyrosin-Aminosäure der Verbindung der Formel (I) [R3 gleich H] zu einer Acylgruppe [R3 gleich C(=O)-(C₁-C₆)-Alkyl)] und/oder der OH-Gruppe an der 3-Hydroxy-Valin-Aminosäure der Verbindung der Formel (I) [R4 gleich H] zu einer Acylgruppe [R4 gleich C(=O)-(C₁-C₆)-Alkyl)] erfolgt nach an sich bekannten Methoden (J. March, Advanced Organic Chemistry, John Wiley & Sons, 4th Edition, 1992), beispielsweise durch Umsetzung mit einem Säurechlorid in Gegenwart einer Base oder mit einem Säureanhydrid.

Die Alkylierung der OH-Gruppe an der 3,5-Dichlor-Tyrosin-Aminosäure der Verbindung der Formel (I) [R3 gleich H] mit einer Alkylgruppe [R3 gleich (C₁-C₆)-Alkyl] und/oder der OH-Gruppe an der 3-Hydroxy-Valin-Aminosäure der Verbindung der Formel (I) [R4 gleich H] mit einer Alkylgruppe [R4 gleich (C₁-C₆)-Alkyl] erfolgt mittels dem Fachmann an sich bekannter Methoden (J. March, Advanced Organic Chemistry, John Wiley & Sons, 4th Edition, 1992), beispielsweise durch Reaktion mit einem (C₁-C₆)-Alkylbromid in Gegenwart einer Base oder im Falle einer Methylierung durch Reaktion mit Methyliodid oder Me₂SO₄.

Eine selektive Differenzierung zwischen der phenolischen OH-Gruppe (R3=H) und der aliphatischen OH-Gruppe (R4=H) erfolgt mittels dem Fachmann an sich bekannter Methoden zur Einführung von Schutzgruppen (T. W. Greene, P. G. M. Wuts, Protective Groups in Organic Synthesis, John Wiley & Sons, 3rd Edition, 1999). Beispielsweise beschreibt Pettus et al. (J. Am. Chem. Soc. 2000, 122, 6160-6168) eine selektive Alkylierung einer phenolischen OH-Gruppe in Gegenwart eines tertiären aliphatischen Alkohols durch Umsetzung mit (C₁-C₆)-Alkylbromid in Gegenwart von K₂CO₃ in Aceton oder durch Umsetzung mit (C₁-C₆)-Alkyl-OH in Gegenwart von (CF₃CO)₂O und CuCl₂-Hydrat in DBU. Eine weitere Möglichkeit zur Differenzierung zwischen der phenolischen OH-Gruppe und der aliphatischen OH-Gruppe erfolgt mittels dem Fachmann an sich bekannter Methoden zur selektiven Entschützung einer Bis-alkylierten [R3 gleich R4 gleich (C₁-C₆)-Alkyl] oder einer Bis-acylierten [R3 gleich R4 gleich C(=O)-(C₁-C₆)-Alkyl)] Verbindung der Formel (I) (T. W. Greene, P. G. M. Wuts, Protective Groups in Organic Synthesis, John Wiley & Sons, 3rd Edition, 1999).

Beispielsweise beschreibt Jones et al. (J. Org. Chem. 2001, 66, 3688-3695) die selektive Entschützung eines tert-Butylsilyl (TBS)-geschützten Phenols in Gegenwart eines TBS-geschützten tertiären Alkohols durch tert-Butylammoniumfluorid (TBAF) bei -20°C. Die phenolische OH-Gruppe (R3 = H) kann ferner derivatisiert zu einer Gruppe -(C₁-C₆)-Alkylen-NH-(C₁-C₆)-Alkyl werden durch Umsetzung mit H₂N-(C₁-C₆)-Alkyl in Gegenwart von Cl-[(C₁-C₆)-Alkyl]-Cl oder Br-[(C₁-C₆)-Alkyl]-Br.

Die Derivatisierung von Verbindungen der Formel (I), in denen X und Y eine Gruppe - O- bilden zu einer Verbindung der Formel (I), in der X und Y unabhängig voneinander OH, O-(C₁-C₆)-Alkyl, NH₂ oder NH-(C₁-C₆)-Alkyl sind, erfolgt mittels dem Fachmann an sich bekannter Methoden (J. March, Advanced Organic Chemistry, John Wiley & Sons, 4th Edition, 1992), beispielsweise durch Reaktion der Epoxidgruppe mit einem (C₁-C₆)-Alkoholat [X gleich OH wenn Y gleich O-(C₁-C₆)-Alkyl, oder Y gleich OH wenn X gleich O-(C₁-C₆)-Alkyl], NH₃ [X gleich OH wenn Y gleich NH₂, oder Y gleich OH wenn X gleich NH₂], oder H₂N-(C₁-C₆)-Alkyl [X gleich OH wenn Y gleich NH-(C₁-C₆)-Alkyl, oder Y gleich OH wenn X gleich NH-(C₁-C₆)-Alkyl].

Ein Isolat des Mikroorganismenstammes ST 201196 wurde bei der Deutschen Sammlung von Mikroorganismen und Zellkulturen GmbH (DSMZ), Mascheroder Weg 1B, 38124 Braunschweig, Deutschland, nach den Regeln des Budapester Vertrages am 11.09.2003 unter der folgenden Nummer hinterlegt. Als Hinterlegungsnummer wurde die folgende Nummer vergeben: DSM 18870.

Die vegetativen Zellen des Stammes ST 201196 (DSM 18870) haben eine charakteristische Stäbchenform. Auf festen Nährböden bildet ST 201196 (DSM 18870) orange-gelbe Fruchtkörper, welche runde Myxosporen enthalten. Die Taxonomie des Stammes ST 201196 kann daher als Myxobakterium sp. beschrieben werden.

Anstelle des Stammes ST 201196 (DSM 18870) können auch dessen Mutanten und/oder Varianten eingesetzt werden, die ein oder mehrere der erfindungsgemäßen Verbindungen synthetisieren.

Eine Mutante ist ein Mikroorganismus, in dem ein oder mehrere Gene des Genoms modifiziert wurden, wobei das Gen oder die Gene funktionell und vererbbar erhalten bleiben, die für die Fähigkeit des Organismus verantwortlich sind, die erfinderische Verbindung zu produzieren.

Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethytmethansulfonat (EMS); 2-Hydroxy-4-methoxy-benzophenon (MOB) oder N-Methyl-N'-nitro-N-nitrosoguanidin (MNNG) erzeugt werden, oder wie von Brock et al. in "Biology of Microorganisms", Prentice Hall, Seite 238-247 (1984) beschrieben.

Eine Variante ist ein Phenotyp des Mikroorganismus. Mikroorganismen haben die Fähigkeit, sich an ihre Umgebung anzupassen und zeigen daher ausgeprägte physiologische Flexibilität. Bei der phenotypischen Anpassung sind alle Zellen des Mikroorganismus involviert, wobei die Art der Veränderung nicht genetisch konditioniert ist und unter veränderten Bedingungen reversibel ist (H. Stolp, Microbial ecology: organismus, habitats, activities. Cambridge University Press, Cambridge, GB, Seite 180, 1988).

Das Screening nach Mutanten und/oder Varianten, die ein oder mehrere der erfindungsgemäßen Verbindungen synthetisieren, erfolgt nach folgendem Schema:
- Lyophilisierung des Fermentationsmediums;
- Extraktion des Lyophilisats mit einem organischen Lösungsmittel
- Extraktion der Verbindung aus dem Kulturfiltrat mit Festphasen
- Analytik mittels HPLC, DC oder durch Testen der biologischen Wirksamkeit.

Die beschriebenen Fermentationsbedingungen gelten für ST 201196 (DSM 18870) sowie für Mutanten und/oder Varianten davon.

Zum Nachweis der antifungischen Aktivität schnellwachsender, aerober Krankheitserreger bedient man sich der Bouillon-Dilutionsmethode (Mikrodilution) nach einer Vorschrift der CLSI (Clinical and Laboratory Standards Institute, M7-A7, Vol. 26, No. 2). Es wurde der IC₅₀-Wert bestimmt. Dies ist die Konzentration eines Wirkstoffes, die notwendig ist, um das Wachstum des Testorganismus *Candida albicans* um 50% zu hemmen.

Die Verbindung der Formel (II) hat gegen Candida albicans einen IC₅₀-Wert von 0,06 µg/ml. Die Verbindung der Formel (IV) hat gegen *Candida albicans* einen IC₅₀-Wert von 0,41 µg/ml.

Gegenstand der Erfindung ist ferner die Verwendung der Verbindung der Formel (I) oder eines physiologisch verträglichen Salzes derselben als Arzneimittel in der Human- oder Tiermedizin, insbesondere zur Behandlung und/oder Prophylaxe von Pilzerkrankungen. Vorzugsweise betrifft die Erfindung die Verwendung einer Verbindung der Formel (I) oder eines physiologisch verträglichen Salzes zur Behandlung von lokalen und/oder systemischen Pilzerkrankungen.

Des weiteren betrifft die vorliegende Erfindung ein Arzneimittel mit einem Gehalt an mindestens einer Verbindung der Formel (I), wobei die Verbindung oder die Verbindungen der Formel (I) als solche in Substanz verabreicht werden kann oder bevorzugt in Mischung mit einem oder mehreren der üblichen pharmakologisch geeigneten Träger- oder Hilfsstoffen.

Die erfindungsgemäßen Verbindungen sind im festen Zustand und in Lösungen im pH-Bereich zwischen 2 und 9, insbesondere 5 und 7, stabil und lassen sich damit in übliche galenische Zubereitungen einarbeiten.

Die erfindungsgemäßen Arzneimittel können oral oder parenteral verabreicht werden, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise pharmakologisch geeignete Träger- oder Hilfsstoffe wie Spreng-, Binde-, Überzugs-, Quellungs-, Gleit- oder Schmiermittel, Geschmacksstoffe, Süßungsmittel oder Lösungsvermittler Verwendung finden, beispielsweise Magnesiumcarbonat, Titandioxid, Laktose, Mannitol und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Naturstoff-Derivate enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, dass man ein oder mehrere der erfindungsgemäßen Verbindungen der Formel (I) optional mit einem oder mehreren der üblichen Träger- oder Hilfsstoffe mischt sowie in eine geeignete Darreichungsform bringt.

Die folgenden Beispiele sollen der näheren Erläuterung der Erfindung dienen.

Prozentangaben beziehen sich auf das Gewicht. Mischungsverhältnisse bei Flüssigkeiten beziehen sich auf das Volumen, wenn keine anderen Angaben gemacht wurden.

### Beispiel 1: Einlagerung bei -135°C von ST 201196 (DSM 18870)

Eine Agarplatte (1 % frische Bäckerhefe; 1 % CaCl₂ x 2 H₂O; 0,477 % HEPES (20 mM); 0,00005 % Cyanocobalamin; 1,8% Agar; pH 7,2) wurde mit dem Stamm ST 201196 (DSM 18870) beimpft und ca. 7-10 Tage bei 30° C inkubiert. Die Zellen dieser Oberflächenkultur wurden mit einem sterilen Spatel von der Agaroberfläche gekratzt, in 1 mL Casitonmedium (1% Casitone (Difco); 0,15 % MgSO₄ x 7H₂O; pH 7,0) in Kryoröhrchen suspendiert und bei -135°C gelagert.

### Beispiel 2: Einlagerung bei -196°C von ST 201196 (DSM 18870)

Eine Agarplatte (1 % frische Bäckerhefe; 1 % CaCl₂ x 2 H₂O; 0,477 % HEPES (20 mM); 0,00005 % Cyanocobalamin; 1,8% Agar; pH 7,2) wurde mit dem Stamm ST 201196 (DSM 18870) beimpft und ca. 7-10 Tage bei 30° C inkubiert. Die Zellen dieser Oberflächenkultur wurden mit einem sterilen Spatel von der Agaroberfläche gekratzt, in 1 mL Casitonmedium (1% Casitone (Difco); 0,15 % MgSO₄ x 7H₂O; pH 7,0) in Kryoröhrchen suspendiert und bei -196°C gelagert.

### Beispiel 3: Herstellung einer Vorkultur im Erlenmeyerkolben von ST 201196 (DSM 18870)

100 mL Nährlösung (1 % frische Bäckerhefe; 1 % CaCl₂ x 2 H₂O; 0,477 % HEPES (20 mM); 0,00005 % Cyanocobalamin; 1,8 % Agar; pH 7,2) in einem sterilen 300 mL Erlenmeyerkolben wurden mit dem Stamm ST 201196 (DSM 18870) beimpft und 7 Tage bei 30°C und 180 UpM auf einer rotierenden Schüttelmaschine inkubiert. Jeweils 10 mL (10 %) dieser Vorkultur wurden anschließend für die Herstellung der Hauptkulturen verwendet.

### Beispiel 4: Herstellung einer flüssigen Hauptkultur ST 201196 (DSM 18870) mit Medium 1

Ein steriler 300 mL Erlenmeyerkolben mit 100 mL der folgenden Nährlösung (1 % Probion F; 0,1 % CaCl₂ x 2 H₂O; 0,2 % MgSO₄ x 7 H₂O; 0,00005 % Cyanocobalamin; 2 % des Adsorberharzes XAD-16, pH 8,4) wurde mit 10 mL (10%) einer Vorkultur (s. Beispiel 3) oder einer auf einer frischen Agarplatte gewachsenen Kultur (1 % frische Bäckerhefe; 1 % CaCl₂ x 2 H₂O; 0,477% HEPES (20 mM); 0,00005 % Cyanocobalamin; 1,8 % Agar; pH 7,2) beimpft und auf einer Schüttelmaschine bei 180 UpM und 30° C inkubiert. Die maximale Produktion der erfindungsgemäßen Substanzen war nach 144 bis 216 Stunden erreicht. Zum Animpfen von 10 bis 200 L Fermentern genügte eine 144-196 Stunden alte Submerskultur (Inokulum 10 %) aus der gleichen Nährlösung wie in Beispiel 3 beschrieben.

### Beispiel 5: Herstellung einer flüssigen Hauptkultur ST 201196 (DSM 18870) mit Medium 2

Ein steriler 300 mL Erlenmeyerkolben mit 100 mL der folgenden Nährlösung (1 % Hafermehl; 0,5 % Glycerin; 0,1 % CaCl₂ x 2 H₂O; 0,2 % MgSO₄ x 7 H₂O; 0,00005 % Cyanocobalamin; 2 % des Adsorberharzes XAD-16, pH 9,0) wurde mit 10 mL (10%) einer Vorkultur (s. Beispiel 3) oder einer auf einer frischen Agarplatte gewachsenen Kultur (1% frische Bäckerhefe; 1% CaCl₂ x 2 H₂O; 0,477% HEPES (20 mM); 0,00005 % Cyanocobalamin; 1,8% Agar; pH 7,2) beimpft und auf einer Schüttelmaschine bei 180 UpM und 30° C inkubiert. Die maximale Produktion der erfindungsgemäßen Substanzen war nach 1.44 bis 216 Stunden erreicht. Zum Animpfen von 10 bis 200 L Fermentern genügte eine 144-196 Stunden alte Submerskultur (Inokulum 10 %) aus der gleichen Nährlösung wie in Beispiel 3 beschrieben.

### Beispiel 6: Herstellung der erfindungsgemäßen Substanzen im Fermenter

Die 1 L und 50 L Fermenter wurden unter folgenden Bedingungen betrieben:

| | |
|---|---|
| Inokulum: | 20 % |
| Nährmedium: | 1 % Hafermehl; 0,5 % Glycerin; 0,1 % Hefeextrakt; 0,1 % CaCl₂ x 2 H₂O; 0,2 % MgSO₄ x 7 H₂O; 0,00005 % Cyanocobalamin, 2 % des Adsorberharzes XAD-16 |
| Inkubationstemperatur: | 30°C |
| Rührergeschwindigkeit: | 200 UpM |
| Belüftung: | 0,6 m³/h |
| pH Regulierung: | keine, vor Sterilisation pH 7.6±0,3 mittels KOH |
| pO₂ Regulierung: | keine |
| Anitschaumzusatz: | 0,05 % Desmophen (Bayer) |
| Laufzeit: | 155 h |

Die pH-Regulierung wurde mit 10% KOH, bzw. 10% H₂SO₄ durchgeführt.

### Beispiel 7: Isolierung der Verbindungen (II) und (III) aus den Schüttelkulturen des Mikroorganismenstammes ST201196 (DSM 18870)

Nach Beendigung der Fermentation des Mikroorganismenstammes ST201196 (DSM 18870) wurde die Kulturbrühe aus Beispiel 4 (60 L Kulturbrühe) filtriert. Die Biomasse mit dem XAD wurde lyophilisiert und anschließend mit Methanol (4 x 5 L) extrahiert. Der Methanolextrakt wurde am Vakuum auf 8 L reduziert und anschließend auf eine vorbereitete Säule aufgetragen, die mit ca 3.0 Liter CHP-20P Material (MCI® Gel, 75-150µ, Mitsubishi Chemical Corporation) gefüllt war. Eluiert wurde mit einem Methanol-Gradienten von 10% nach 95%. Der Säulendurchfluss (120 ml/min) wurde fraktioniert aufgefangen (1 L Fraktionen). Die Fraktionen 11 bis 14 wurden vereinigt, das Lösungsmittel am Rotavapor entfernt und anschließend wurde der Fraktions-Pool lyophilisiert (Ausbeute ~ 0.9 g).

### Beispiel 8: Vortrennung der Verbindungen (II) und (III) durch RP-18 Chromatographie

Der Fraktions-Pool 11-14 aus Beispiel 7 wurde in 100 ml Methanol gelöst und auf eine Phenomenex Luna® 10µ C18 (2) Säule (Dimension: 250 mm x 50 mm) mit Vorsäule Luna® 10µ C18 (2) (Dimension: 60 mm x 21.2 mm) aufgetragen und mit einem Gradienten von 5% bis 95% Acetonitril in Wasser über 40 min eluiert (0.1 % Ammoniumacetat, pH 4.6 eingestellt mit Essigsäure). Der Durchfluss betrug 190 ml/min, die Fraktionsgröße 190 ml. Die Fraktionen 28-29 und 31 wurden anschließend weiter aufgearbeitet.

### Beispiel 9: Reinigung von Verbindung (II)

Fraktion 31 aus Beispiel 8 wurde zunächst lyophilisiert (Ausbeute ~ 98 mg), anschließend in 50 ml Methanol gelöst und nochmals mittels HPLC an einer Phenomenex Luna® Axia 5 µm C18 (2) Säule (Dimension: 100 mm x 30 mm) mit Vorsäule XTerra® Prep MS C18 10 µm (Waters, Dimension: 19 x 10 mm) aufgereinigt. Eluiert wurde mit einem Gradienten von 5% nach 95% Acetonitril in Wasser über 40 min (mit Zusatz von 0.1 % Ammoniumacetat, pH 4.6 eingestellt mit Essigsäure). Der Säulendurchfluss (50 ml/min) wurde nach UV fraktioniert aufgefangen. Fraktion 4 bis 14 enthielt die Verbindung der Formel (II) und ergab nach Lyophilisieren 38 mg (Reinheit > 95%).

### Beispiel 10: Charakterisierung der Verbindung der Formel (II)

Farbloser Feststoff, Kristalle aus Acetonitril / Wasser
UV: 208, 232, 286 nm
ESI-MS: MW = 815.3312
Summenformel: C₄₂H₅₅Cl₂N₃O₉
Drehwert (MeOH): -0,19°, α_{D}= -38°

**Tabelle 1: NMR-chemische Verschiebungen des Makrolaktons (II); c = 3 mg/ml in d₆-DMSO bei 300 K.**

| Position | δ (13^{C}) | δ (¹H) |
|---|---|---|
| 1 | 126.87 | 7.251 |
| 2 (2C) | 127.71 | 7.322 |
| 3 | 125.89 | 7.566 |
| 4 | 139.90 | - |
| 5 | 78.78 | 5.898 |
| 6 | 41.82 | 2.635 |
| 6-Me | 9.50 | 0.961 |
| 7 | 138.29 | 6.175 |
| 8 | 124.58 | 6.379 |
| 9 | 128.83 | 6.116 |
| 10 | 133.27 | - |
| 10-Me | 10.62 | 1.696 |
| 11 | 83.90 | 3.619 |
| 11-OMe | 55.08 | 3.094 |
| 12 | | 2.115 |
| | 30.50 | 1.456 |
| 13 | 57.97 | 2.633 |
| 14 | 61.26 | - |
| 14-Me | 14.81 | 1.427 |
| 15 | 169.23 | - |
| 16 N-Me | 29.59 | 2.979 |
| 17 α | 59.11 | 4.474 |
| β | 30.94 | 1.704 |
| β-Me | 14.14 | 0.348 d |
| Y | | 1.218 |
| | 24.15 | 0.863 |
| δ | 9.99 | 0.770 t |
| 18 CO | 168.59 | - |
| 19 NH | | 8.014 |
| 20 α | 52.67 | 4.686 |
| β | | 2.806 |
| | 37.02 | 2.483 |
| Y | (136.8) | - |
| δ | 129.55 | 7.345 |
| ε | 121.50 | - |
| ϕ | 147.65 | - |
| ϕ-OH | | 9.88 br |
| 21 CO | 170.43 | - |
| 22 NH | | 8.596 |
| 23 α | 59.11 | 4.677 |
| β | 71.84 | - |
| β-OH | | 5.110 |
| Y | 24.36 | 1.037 |
| Y' | 28.14 | 1.142 |
| 24 CO | 170.55 | - |

### Beispiel 11: Reinigung von Verbindung (III)

Fraktion 28-29 (380 ml) aus Beispiel 8 wurde nochmals mittels HPLC an einer Waters XTerra® 10 µm C18 Säule (Dimension: 100 mm x 30 mm) mit Vorsäule XTerra® Prep MS C18 10 µm (Waters, Dimension: 19 x 10 mm) aufgereinigt. Eluiert wurde mit einem Gradienten von 10% nach 95% Acetonitril in Wasser über 40 min (mit Zusatz von 10% Ameisensäure, pH = 2.0). Der Säulendurchfluss (70 ml/min) wurde nach UV fraktioniert aufgefangen. Fraktion 45 bis 47 enthielt die Verbindung der Formel (III) und ergab nach Lyophilisieren ~ 5.4 mg (Reinheit > 50%).

### Beispiel 12: Charakterisierung der Verbindung (III)

Farbloser Feststoff
UV: 204, 232, 286 nm
ESI-MS: MW = 799.3002
Summenformel: C₄₂H₅₆ClN₃O₉

**Tabelle 2: NMR-chemische Verschiebungen des Makrolaktons (III); c = 5 mg/ml in d₆-DMSO bei 300 K.**

| Position | δ (¹³C) | δ (¹H) |
|---|---|---|
| 1 | 126.88 | 7.252 |
| 2 (2C) | 127.71 | 7.331 |
| 3 | 125.93 | 7.566 |
| 4 | 139.91 | - |
| 5 | 78.76 | 5.896 |
| 6 | 41.84 | 2.659 br. |
| 6-Me | 9.59 | 0.958 |
| 7 | 138.25 | 6.171 |
| 8 | 124.64 | 6.375 |
| 9 | 128.79 | 6.114 |
| 10 | 133.29 | - |
| 10-Me | 10.68 | 1.692 |
| 11 | 83.86 | 3.620 |
| 11-OMe | 55.09 | 3.090 |
| 12 | | 2.113 |
| | 30.52 | 1.470 |
| 13 | 57.98 | 2.642 |
| 14 | 61.23 | - |
| 14-Me | 14.81 | 1.431 |
| 15 | 169.27 | - |
| 16 N-Me | 29.63 | 2.981 |
| 17 α | 59.08 | 4.471 |
| β | 31.04 | 1.693 |
| β-Me | 14.80 | 0.315 d. |
| Y | 24.16 | 1.221 |
| | | 0.862 |
| δ | 9.99 | 0.773 t |
| 18 CO | 168.57 | - |
| 19 NH | | 7.995 |
| 20 α | 52.68 | 4.716 |
| β | | 2.824 |
| | 37.26 | 2.488 |
| γ | 129.25 | - |
| δ | 129.12 | 7.069 |
| δ' | 130.67 | 7.335 |
| ε | 115.83 | 6.767 |
| ε' | 118.85 | - |
| ϕ | 151.40 | - |
| ϕ-OH | | n.d. |
| 21 CO | 170.73 | - |
| 22 NH | | 8.636 |
| 23 α | 59.20 | 4.674 |
| β | 71.85 | - |
| β-OH | | n.d |
| γ | 24.41 | 1.035 |
| γ' | 28.15 | 1.142 |
| 24 CO | 170.54 | - |

### Beispiel 13: Synthese von Verbindung (IV)

Verbindung (II) (80 mg, 0.098 mmol) wurde in 5 ml Acetonitril gelöst und bei Raumtemperatur mit Kaliumcarbonat (27 mg, 0.196 mmol) and Methyliodid (70 mg, 0.490 mmol) versetzt. Das Gemisch wurde anschließend 12 h bei 50°C gerührt. Die Lösung wurde filtriert und mittels HPLC an einer Phenomenex Luna® Axia 5 µm C18 (2) Säule (Dimension: 100 mm x 30 mm) mit Vorsäule XTerra® Prep MS C18 10 µm (Waters, Dimension: 19 x 10 mm) aufgereinigt. Eluiert wurde mit einem Gradienten von 5% nach 95% Acetonitril in Wasser über 40 min (mit Zusatz von 0.1% Ammoniumacetat, pH 4.6, eingestellt mit Essigsäure). Der Säulendurchfluss (50 ml/min) wurde nach UV fraktioniert aufgefangen. Fraktion 4 und 5 enthielt die gewünschte Verbindung der Formel (IV) und ergab nach Lyophilisieren 50 mg (Ausbeute: 61%, Reinheit > 95%).

### Beispiel 14: Charakterisierung der Verbindung der Formel (IV)

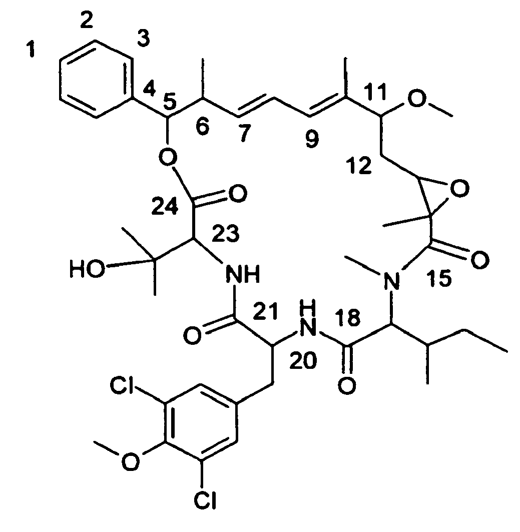

Farbloser Feststoff, Kristalle aus Acetonitril / Wasser
UV: 235, 286 nm
MW = 830.85
Summenformel: C₄₃H₅₇Cl₂N₃O₉

**Tabelle 3: NMR-chemische Verschiebungen der Verbindung der Formel (IV); c = 3 mg/ml in d₆-DMSO bei 300 K.**

| Position | δ (¹³C) | δ (¹H) |
|---|---|---|
| 1 | 126.88 | 7.267 |
| 2 (2C) | 127.72 | 7.332 |
| 3 (2C) | 125.88 | 7.568 |
| 4 | 139.87 | - |
| 5 | 78.78 | 5.903 |
| 6 | 41.81 | 2.660 |
| 6-Me | 9.593 | 0.969 |
| 7 | 138.23 | 6.192 |
| 8 | 124.60 | 6.385 |
| 9 | 128.78 | 6.124 |
| 10 | 133.27 | - |
| 10-Me | 10.65 | 1.700 |
| 11 | 83.88 | 3.624 |
| 11-OMe | 55.08 | 3.103 |
| 12 | | 2.130 |
| | 30.46 | 1.470 |
| 13 | 57.94 | 2.637 |
| 14 | 61.26 | - |
| 14-Me | 14.82 | 1.437 |
| 15 | 169.23 | - |
| 16 N-Me | 29.55 | 2.984 |
| 17 α | 59.04 | 4.471 |
| β | 30.87 | 1.715 |
| β-Me | 14.20 | 0.284 |
| γ | | 1.216 |
| | 24.10 | 0.884 |
| δ | 9.95 | 0.775 |
| 18 CO | 168.53 | - |
| 19 NH | | 8.103 |
| 20 α | 52.37 | 4.771 |
| β | | 2.889 |
| | 37.19 | 2.569 |
| γ | 136.23 | - |
| δ | 130.29 | 7.511 |
| ε | 127.33 | - |
| ϕ | 149.79 | - |
| ϕ-OMe | 60.29 | 3.742 |
| 21 CO | 170.19 | - |
| 22 NH | | 8.620 |
| 23 α | 59.13 | 4.691 |
| β | 71.80 | - |
| β-OH | | 5.161 |
| γ | 24.38 | 1.047 |
| γ' | 28.12 | 1.153 |
| 24 CO | 170.55 | - |

### Beispiel 15: Synthese der Verbindungen (V) und (VI)

Verbindung (II) (30 mg, 0.037 mmol) wurde in 10 ml 1,2-Dichlorethan gelöst und bei Raumtemperatur mit Isobutylamin (500 µl, 5.03 mmol) versetzt. Das Gemisch wurde 48 h bei 70 °C gerührt, anschließend filtriert und mittels HPLC an einer Phenomenex Luna® Axia 5 µm C18 (2) Säule (Dimension: 100 mm x 30 mm) mit Vorsäule XTerra® Prep MS C18 10 µm (Waters, Dimension: 19 x 10 mm) aufgereinigt. Eluiert wurde mit einem Gradienten von 5% nach 95% Acetonitril in Wasser über 40 min (mit Zusatz von 0.1 % Ammoniumacetat, pH 4.6 eingestellt mit Essigsäure). Der Säulendurchfluss (50 ml/min) wurde nach UV fraktioniert aufgefangen. Fraktion 70 enthielt die beiden Verbindungen der Formel (V) und (VI) und ergab nach Lyophilisieren 3 mg der beiden Verbindungen im Verhältnis von 55:45.

### Beispiel 16: Charakterisierung der Verbindung der Formel (V)

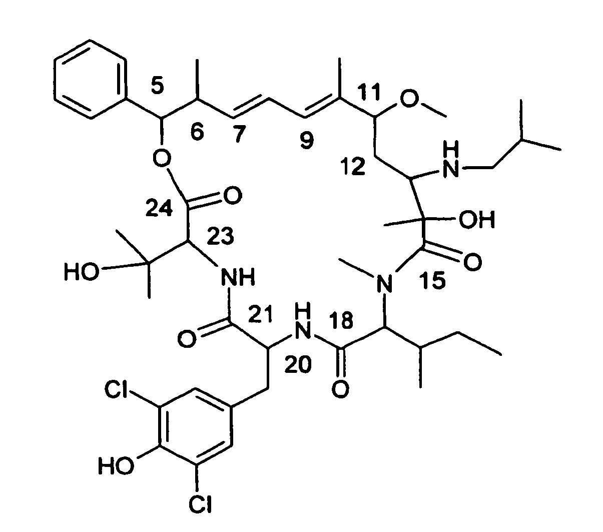

MW = 889.97
Summenformel: C₄₆H₆₆Cl₂N₄O₉

**Tabelle 4: NMR-chemische Verschiebungen der Verbindung der Formel (V); c = 3 mg/ml in d₆-DMSO bei 300 K.**

| Position | δ C(¹³C) | s (¹H) |
|---|---|---|
| 1 | 126.94 | 7.244 |
| 2 (2C) | 127.86 | 7.340 |
| 3 (2C) | 125.82 | 7.507 |
| 4 | 139.29 | - |
| 5 | 78.48 | 5.768 |
| 6 | 41.63 | 2.679 |
| 6-Me | 12.06 | 0.880 |
| 7 | 134.90 | 5.776 |
| 8 | 126.58 | 6.483 |
| 9 | 124.04 | 5.901 |
| 10 | 137.71 | - |
| 10-Me | 13.72 | 1.690 |
| 11 | 82.17 | 3.679 |
| 11-OMe | 55.06 | 3.083 |
| 12 | 37.38 | 1.379 1.265 |
| 13 | 61.04 | 2.927 |
| 13-NH | | n.d. |
| 13-iBu-1 | | 2.435 |
| | 58.81 | 2.460 |
| 13-iBu-2 | 28.98 | 1.624 |
| 13-iBu-3 | 20.66 | 0.903 |
| 13-iBu-3 | 20.66 | 0.896 |
| 14 | 61.28 | - |
| 14-OH | | n.d. |
| 14-Me | 26.44 | 1.302 |
| 15 | 174.64 | - |
| 16 | - | - |
| 16 N-Me | 28.98 | 3.190 |
| 17 α | 59.71 | 4.750 |
| β | 32.15 | 1.678 |
| β-Me | 14.15 | 0.208 |
| γ | | 0.814 |
| | 23.70 | 1.207 |
| δ | 10.17 | 0.724 |
| 18 CO | 168.46 | - |
| 19 NH | | 8.242 |
| 20 α | 52.74 | 4.927 |
| β | | 2.646 |
| | 37.96 | 2.843 |
| γ | 122.4 | - |
| δ | 129.59 | 7.358 |
| ε | 121.57 | - |
| ϕ | 147.54 | - |
| ϕ-OH | | n.d. br. |
| 21 CO | 170.72 | - |
| 22 NH | | 8.451 |
| 23 α | 59.99 | 4.598 |
| β | 71.16 | - |
| β-OH | | 5.120 |
| γ | 25.13 | 1.092 |
| γ' | 28.09 | 1.191 |
| 24 CO | 170.72 | - |

### Beispiel 17: Charakterisierung der Verbindung der Formel (VI)

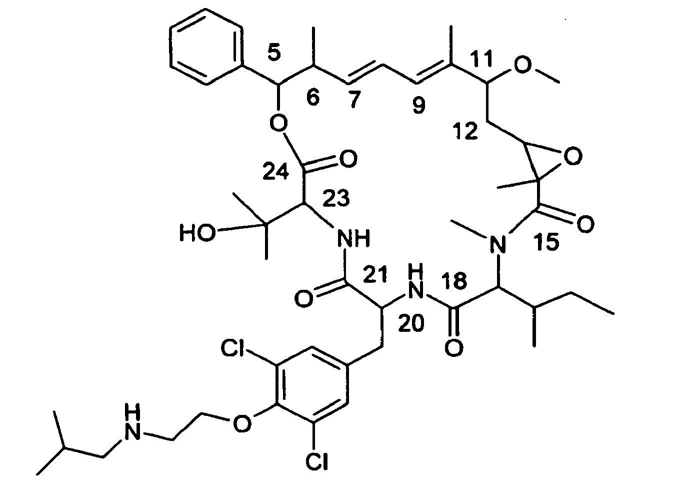

MW = 916.00
Summenformel: C₄₈H₆₈Cl₂N₄O₉

**Tabelle 5: NMR-chemische Verschiebungen der Verbindung der Formel (VI); c = 3 mg/ml in d₆-DMSO bei 300 K.**

| Position | δ (¹³C) | δ (¹H) |
|---|---|---|
| 1 | 127.00 | 7.267 |
| 2 (2C) | 127.72 | 7.330 |
| 3 (2C) | 125.89 | 7.564 |
| 4 | 139.88 | - |
| 5 | 78.79 | 5.900 |
| 6 | 41.82 | 2.660 |
| 6-Me | 9.53 | 0.968 |
| 7 | 138.25 | 6.186 |
| 8 | 124.61 | 6.383 |
| 9 | 128.82 | 6.123 |
| 10 | 133.28 | - |
| 10-Me | 10.67 | 1.700 |
| 11 | 83.89 | 3.626 |
| 11-OMe | 54.09 | 3.097 |
| 12 | | 2.125 |
| | 30.47 | 1.463 |
| 13 | 57.95 | 2.631 |
| 14 | 61.27 | - |
| 14-Me | 14.83 | 1.436 |
| 15 | 169.25 | - |
| 16 | - | - |
| 16 N-Me | 29.36 | 2.979 |
| 17 α | 59.06 | 4.467 |
| β | 30.89 | 1.701 |
| β-Me | 14.26 | 0.274 |
| γ | | 0.864 |
| | 24.13 | 1.217 |
| δ | 9.97 | 0.773 |
| 18 CO | 168.55 | - |
| 19 NH | | 8.104 |
| 20 α | 52.37 | 4.758 |
| β | | 2.555 |
| | 37.10 | 2.887 |
| γ | 136.04 | - |
| δ | 130.32 | 7.506 |
| ε | 127.34 | - |
| ϕ | 148.96 | - |
| ϕ-1 | 72.94 | 3.952 |
| ϕ-2 | 48.79 | 2.864 |
| NH | | n.d. br. |
| iBu-1 | 57.13 | 2.379 |
| iBu-2 | 27.94 | 1.662 |
| iBu-3 | 20.52 | 0.872 |
| iBu-3 | 20.52 | 0.852 |
| 21 CO | 170.21 | - |
| 22 NH | | 8.612 |
| 23 α | 59.14 | 4.680 |
| β | 71.81 | - |
| β-OH | | 5.161 |
| γ | 24.38 | 1.042 |
| γ' | 28.13 | 1.149 |
| 24 CO | 170.56 | - |

### Beispiel 18: Bestimmung der antifungischen Aktivität gegen Candida albicans

Eine Stammlösung von 1000 µg/ml Wirkstoff [beispielsweise der Verbindung der Formel (II) oder der Verbindung der Formel (IV)] in Methanol wurde hergestellt. Der Teststamm (*Candida albicans* FH 2173) wurde bei -80°C gelagert. Das Inokulum wurde aus einer frischen, flüssigen Vorkultur hergestellt. Die Vorkultur wurde aus einem Kügelchen von dem bei -80°C gelagerten Material und 30 ml Nährmedium (Sabourad-Dextrose Broth, Difco) hergestellt und bei 37°C und 180 Umdrehungen pro Minute für 24 Stunden inkubiert. Das Inokulum ist so einzusehen, dass nach Inokulation des Testbehältnisses die erforderliche Anzahl an koloniebildenden Einheiten erreicht wird. Hierzu wurde das Inokulum mittels eines Photometers bei einer Wellenlänge von 590 nm auf einen Wert von 10⁷ CFU/ml eingestellt (CFU: colony forming units). Nach dem Einstellen des Inokulums wurde die Suspension mit Nährlösung (Mueller Hinton Broth, Difco) 1:100 verdünnt. Innerhalb von 15 Minuten nach Herstellung des Inokulums wurde die Mikrotiterplatte beimpft. Die genaue Koloniezahl wurde mittels Oberflächenkultur bestimmt. Mit der Stammlösung des Wirkstoffes und dem Nährmedium (Mueller Hinton Broth, Difco) wurde zuvor auf der Mikrotiterplatte eine Verdünnungsreihe erstellt. Der Wirkstoff lag in einem Volumen von 20 µl vor und wurde mit 20 µl Inokulum versetzt, so dass man ein Gesamttestvolumen von 40 µl erhielt. Die beimpften Mikrotiterplatten wurden anschließend mit einem Deckel verschlossen und bei 37°C in 5% CO₂ und 95 % Luftfeuchtigkeit für 20 Stunden inkubiert. Für jeden Test wurden auf einer 384er Mikrotiterplatte eine Wirkstoff-freie Kontrolle, eine Steril-Kontrolle und als Referenzsubstanzen Ciprofloxacin und Nystatin mitgetestet. Die Ablesung der Mikrotiterplatten erfolgt mit Hilfe eines Photometers bei einer Wellenlänge von 590 nm durch Messung der Absorption. Die IC₅₀-Werte als die Konzentration des Wirkstoffes, die notwendig ist, um das Wachstum des Testorganismus *Candida albicans* um 50% zu hemmen, wurden anschließend nach einem Standardverfahren aus den Werten der Verdünnungsreihe berechnet.

## Patentansprüche

1. Verbindung der Formel (I), 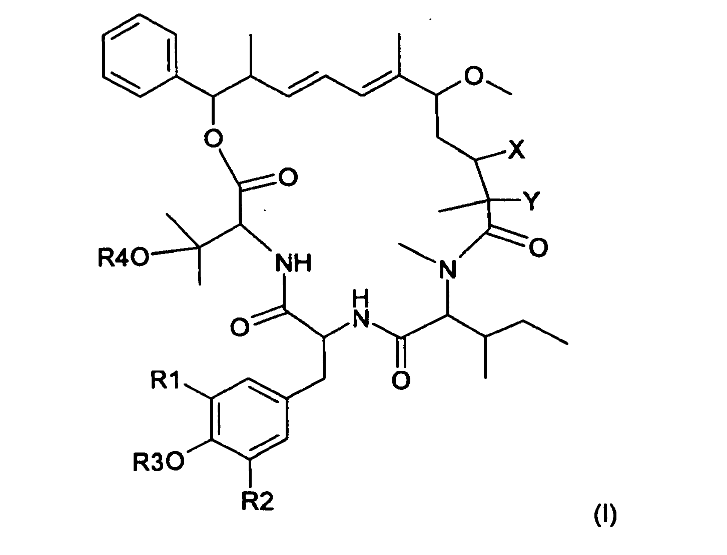 worin
X und Y unabhängig voneinander OH, O-(C₁-C₆)-Alkyl, NH₂ oder NH-(C₁-C₆)-Alkyl sind, oder X und Y zusammen eine Gruppe -O- bilden,
R1 und R2 unabhängig voneinander Cl oder H sind,
R3 H, (C₁-C₆)-Alkyl, C(=O)-(C₁-C₆)-Alkyl oder (C₁-C₆)-Alkylen-NH-(C₁-C₆)-alkyl bedeutet, und
R4 H, (C₁-C₆)-Alkyl oder C(=O)-(C₁-C₆)-Alkyl bedeutet,
oder ein physiologisch verträgliches Salz einer Verbindung der Formel (I).

2. Verbindung der Formel (I) gemäß Anspruch 1, worin X und Y zusammen eine Gruppe -O- bilden.

3. Verbindung der Formel (I) gemäß einem der Ansprüche 1 bis 2, wobei R3 und R4 unabhängig voneinander H, (C₁-C₆)-Alkyl oder C(=O)-(C₁-C₆)-Alkyl sind.

4. Verbindung der Formel (I) gemäß einem der Ansprüche 1 bis 3, worin X und Y zusammen eine Gruppe -O- bilden, R1 und R2 unabhängig voneinander Cl oder H sind, und R3 und R4 unabhängig voneinander H, (C₁-C₆)-Alkyl oder C(=O)-(C₁-C₆)-Alkyl bedeuten.

5. Verbindung der Formel (I) gemäß einem der Ansprüche 1 bis 4, wobei R1 und R2 gleich Cl sind.

6. Verbindung der Formel (I) gemäß einem der Ansprüche 1 bis 4, wobei R1 gleich Cl und R2 gleich H ist.

7. Verbindung der Formel (I) gemäß einem der Ansprüche 1 bis 6, wobei R3 und R4 gleich H sind.

8. Verbindung der Formel (I) gemäß einem der Ansprüche 1 bis 7, wobei R1, R2, R3 und R4 gleich H sind.

9. Verwendung einer Verbindung der Formel (I) oder eines physiologisch verträglichen Salzes davon gemäß einem der Ansprüche 1 bis 8 zur Herstellung eines Arzneimittels.

10. Verwendung einer Verbindung der Formel (I) oder eines physiologisch verträglichen Salzes davon gemäß einem der Ansprüche 1 bis 8 zur Herstellung eines Arzneimittels zur Behandlung und/oder Prophylaxe von Pilzerkrankungen.

11. Arzneimittel mit einem Gehalt an mindestens einer Verbindung der Formel (I) gemäß einem der Ansprüche 1 bis 8.

12. Verfahren zur Herstellung einer Verbindung der Formel (I), 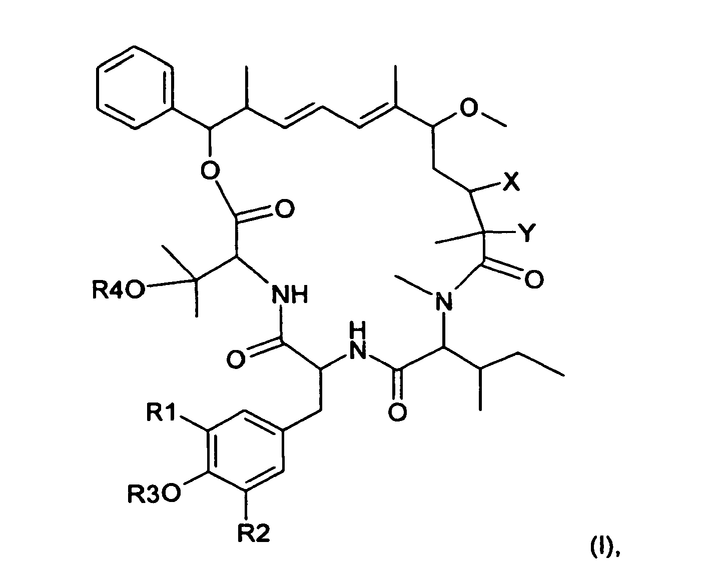 worin
X und Y unabhängig voneinander OH, O-(C₁-C₆)-Alkyl, NH₂ oder NH-(C₁-C₆)-Alkyl sind, oder X und Y zusammen eine Gruppe -O- bilden,
R1 und R2 unabhängig voneinander Cl oder H sind,
R3 H, (C₁-C₆)-Alkyl, C(=O)-(C₁-C₆)-Alkyl oder (C₁-C₆)-Alkylen-NH-(C₁-C₆)-alkyl bedeutet, und
R4 H, (C₁-C₆)-Alkyl oder C(=O)-(C₁-C₆)-Alkyl bedeutet,
oder ein physiologisch verträgliches Salz einer Verbindung der Formel (I),
**dadurch gekennzeichnet, dass**
1. der Stamm ST 201196 (DSM 18870) oder einer seiner Varianten und/oder Mutanten unter geeigneten Bedingungen in einem Kulturmedium fermentiert wird, das eine Cl-Quelle enthält, bis sich eine oder mehrere der Verbindungen der Formel (I) in dem Kulturmedium anhäuft und
2. eine Verbindung der Formel (I) aus dem Kulturmedium isoliert wird, und
3. die Verbindung der Formel (I) gegebenenfalls derivatisiert und/oder in ein physiologisch verträgliches Salz umgewandelt wird.

13. Verfahren gemäß Anspruch 12, wobei in der Verbindung der Formel (I) X und Y zusammen eine Gruppe -O- bilden, R1 und R2 unabhängig voneinander Cl oder H sind, und R3 und R4 unabhängig voneinander H, (C₁-C₆)-Alkyl oder C(=O)-(C₁-C₆)-Alkyl bedeuten.

14. Mikroorganismenstamm ST 201196 (DSM 18870).

## Claims

1. A compound of the formula (I), 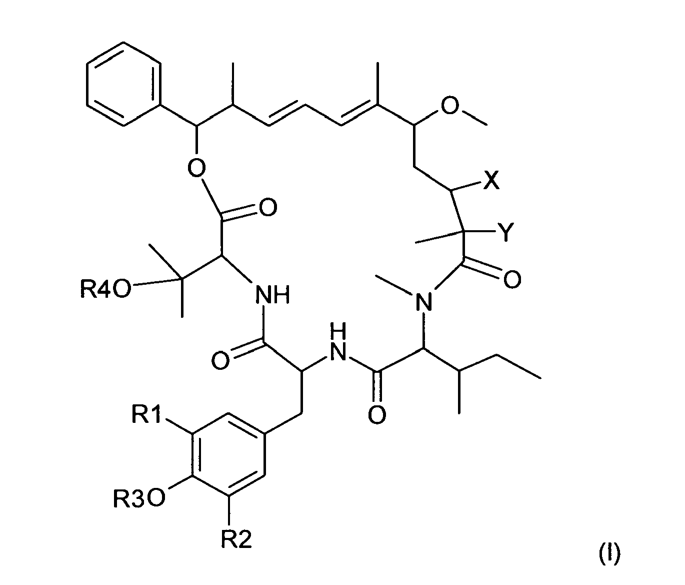 wherein
X and Y independently of one another are OH, O-(C₁-C₆)-alkyl, NH₂ or NH-(C₁-Cg)-alkyl, or X and Y together form a group -O-,
R1 and R2 independently of one another are Cl or H,
R3 is H, (C₁-C₆)-alkyl, C(=O)-(C₁-C₆)-alkyl or (C₁-C₆)-alkylene-NH-(C₁-C₆)-alkyl, and
R4 is H, (C₁-C₆)-alkyl or C(=O)-(C₁-C₆)-alkyl,
or a physiologically tolerable salt of a compound of the formula (I).

2. A compound of the formula (I) as claimed in claim 1, wherein X and Y together form a group -O-.

3. A compound of the formula (I) as claimed in one of claims 1 to 2, where R3 and R4 independently of one another are H, (C₁-C₆)-alkyl or C(=O)-(C₁-C₆)-alkyl.

4. A compound of the formula (I) as claimed in one of claims 1 to 3, wherein X and Y together form a group -O-, R1 and R2 independently of one another are Cl or H, and R3 and R4 independently of one another are H, (C₁-C₆)-alkyl or C(=O)-(C₁-C₆)-alkyl.

5. A compound of the formula (I) as claimed in one of claims 1 to 4, where R1 and R2 are equal to Cl.

6. A compound of the formula (I) as claimed in one of claims 1 to 4, where R1 is equal to Cl and R2 is equal to H.

7. A compound of the formula (I) as claimed in one of claims 1 to 6, where R3 and R4 are equal to H.

8. A compound of the formula (I) as claimed in one of claims 1 to 7, where R1, R2, R3 and R4 are equal to H.

9. The use of a compound of the formula (I) or of a physiologically tolerable salt thereof as claimed in one of claims 1 to 8 for the preparation of a medicament.

10. The use of a compound of the formula (I) or of a physiologically tolerable salt thereof as claimed in one of claims 1 to 8 for the preparation of a medicament for the treatment and/or prophylaxis of fungal disorders.

11. A medicament containing at least one compound of the formula (I) as claimed in one of claims 1 to 8.

12. A process for the preparation of a compound of the formula (I), 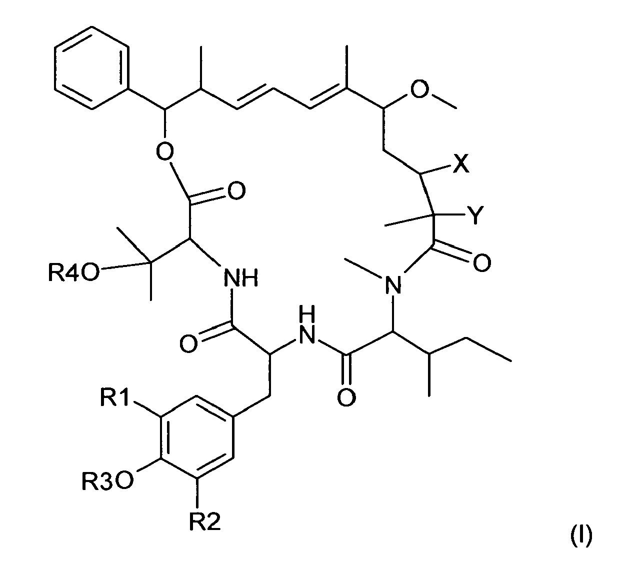 wherein
X and Y independently of one another are OH, O-(C₁-C₆)-alkyl, NH₂ or NH-(C₁-C₆)-alkyl, or X and Y together form a group -O-,
R1 and R2 independently of one another are Cl or H,
R3 is H, (C₁-C₆)-alkyl, C(=O)-(C₁-C₆)-alkyl or (C₁-C₆)-alkylene-NH-(C₁-C₆)-alkyl, and
R4 is H, (C₁-C₆)-alkyl or C(=O)-(C₁-C₆)-alkyl,
or a physiologically tolerable salt of a compound of the formula (I),
which comprises
1. fermenting the strain ST 201196 (DSM 18870) or one of its variants and/or mutants under suitable conditions in a culture medium which contains a Cl source, until one or more of the compounds of the formula (I) accumulates in the culture medium and
2. isolating a compound of the formula (I) from the culture medium, and
3. optionally derivatizing the compound of the formula (I) and/or converting it into a physiologically tolerable salt.

13. The process as claimed in claim 12, where in the compound of the formula (I) X and Y together form a group -O-, R1 and R2 independently of one another are Cl or H, and R3 and R4 independently of one another are H, (C₁-C₆)-alkyl or C(=O)-(C₁-C₆)-alkyl.

14. The microorganism strain ST 201196 (DSM 18870).

## Revendications

1. Composé de formule (I) 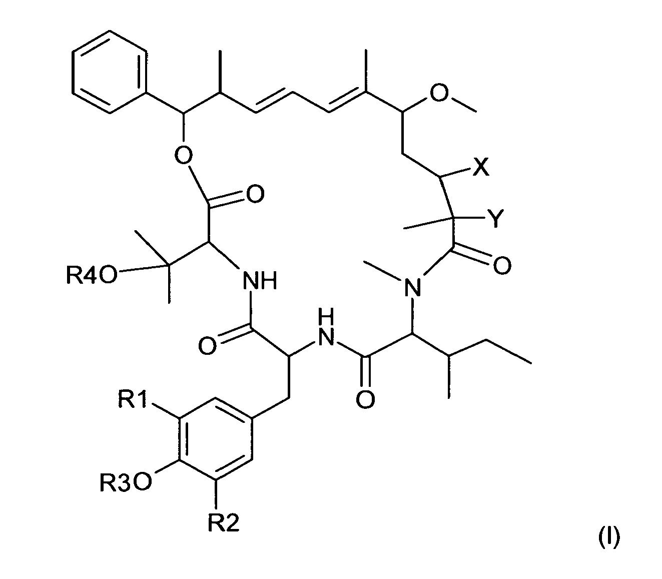 où
X et Y représentent, indépendamment l'un de l'autre, OH, O-(C₁-C₆)-alkyle, NH₂ ou NH-(C₁-C₆)-alkyle, ou X et Y forment ensemble un groupe -O-,
R1 et R2 représentent, indépendamment l'un de l'autre, Cl ou H,
R3 signifie H, (C₁-C₆)-alkyle, C(=O)-(C₁-C₆)-alkyle ou (C₁-C₆)-alkylène-NH-(C₁-C₆)-alkyle, et
R4 signifie H, (C₁-C₆)-alkyle ou C(=O)-(C₁-C₆)-alkyle,
ou un sel physiologiquement acceptable d'un composé de formule (I).

2. Composé de formule (1), selon la revendication 1, où X et Y forment ensemble un groupe -O-.

3. Composé de formule (I) selon l'une quelconque des revendications 1 à 2, où R3 et R4 représentent, indépendamment l'un de l'autre, H, (C₁-C₆)-alkyle ou C(=O)-(C₁-C₆)-alkyle.

4. Composé de formule (1) selon l'une quelconque des revendications 1 à 3, où X et Y forment ensemble un groupe -O-, R1 et R2 représentent, indépendamment l'un de l'autre, Cl ou H et R3 et R4 signifient, indépendamment l'un de l'autre, H, (C₁-C₆)-alkyle ou C(=O)-(C₁-C₆)-alkyle.

5. Composé de formule (I) selon l'une quelconque des revendications 1 à 4, où R1 et R2 représentent Cl.

6. Composé de formule (I) selon l'une quelconque des revendications 1 à 4, où R1 représente Cl et R2 représente H.

7. Composé de formule (I) selon l'une quelconque des revendications 1 à 6, où R3 et R4 représentent H.

8. Composé de formule (I) selon l'une quelconque des revendications 1 à 7, où R1, R2, R3 et R4 représentent H.

9. Utilisation d'un composé de formule (I) ou d'un sel physiologiquement acceptable de celui-ci selon l'une quelconque des revendications 1 à 8 pour la préparation d'un médicament.

10. Utilisation d'un composé de formule (I) ou d'un sel physiologiquement acceptable de celui-ci selon l'une quelconque des revendications 1 à 8 pour la préparation d'un médicament destiné au traitement et/ou à la prophylaxie de mycoses.

11. Médicament contenant une teneur en au moins un composé de formule (I) selon l'une quelconque des revendications 1 à 8.

12. Procédé pour la préparation d'un composé de formule (I) 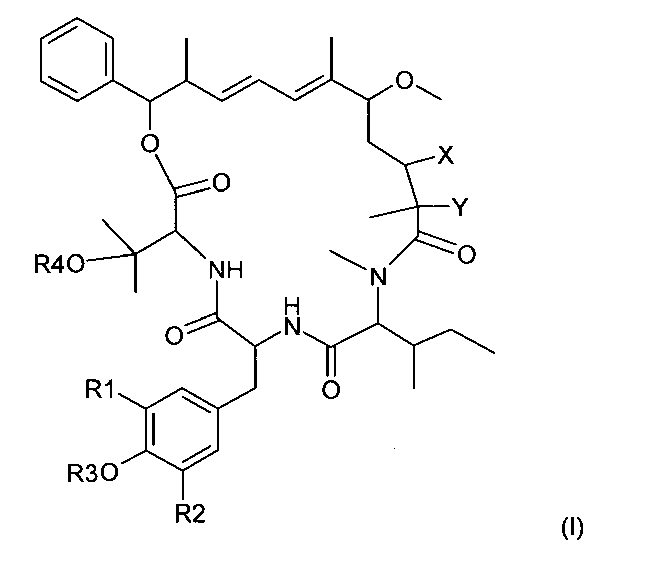 où
X et Y représentent, indépendamment l'un de l'autre, OH, O-(C₁-C₆)-alkyle, NH₂ ou NH-(C₁-C₆)-alkyle, ou X et Y forment ensemble un groupe -O-,
R1 et R2 représentent, indépendamment l'un de l'autre, Cl ou H,
R3 signifie H, (C₁-C₆)-alkyle, C(=O)-(C₁-C₆)-alkyle ou (C₁-C₆)-alkylène-NH-(C₁-C₆)-alkyle, et
R4 signifie H, (C₁-C₆)-alkyle ou C(=O)-(C₁-C₆)-alkyle,
ou d'un sel physiologiquement acceptable d'un composé de formule (I),
**caractérisé en ce que**
1. la souche ST 201196 (DSM 18870) ou une de ses variantes et/ou un de ses mutants est fermenté(e) dans des conditions appropriées dans un milieu de culture, qui contient une source de CI, jusqu'à ce qu'un ou plusieurs des composés de formule (I) s'accumule(nt) dans le milieu de culture et
2. un composé de formule (I) est isolé du milieu de culture, et
3. le composé de formule (I) est le cas échéant dérivé et/ou transformé en un sel physiologiquement acceptable.

13. Procédé selon la revendication 12, où, dans le composé de formule (I), X et Y forment ensemble un groupe -O-, R1 et R2 représentent, indépendamment l'un de l'autre, Cl ou H et R3 et R4 signifient, indépendamment l'un de l'autre, H, (C₁-C₆)-alkyle ou C(=O)-(C₁-C₆)-alkyle.

14. Souche de microorganismes ST 201196 (DSM 18870).
